# EUROPEAN PATENT APPLICATION

(11) **EP 4 748 407 A1**
(43) Date of publication of application: **27.05.2026**
(21) Application number: 25218072.4
(22) Date of filing: 24.11.2025
(51) Int. Cl.: A61M 1/16, A61M 1/36

(54) **ELECTRIC BLOOD HEATER FOR BLOOD OXYGENATOR**

(30) Priority: 25.11.2024 US 202463724621 P
(71) Applicant: Breethe, Inc., Halethorpe, MD 21227 (US)
(72) Inventor: WENHOLZ, Brian, Massachusetts, 01923 (US); UNDERWOOD, Thomas, Massachusetts, 01923 (US)
(74) Representative: ABG Intellectual Property Law, S.L.

(57) **Abstract**

An extracorporeal membrane oxygenation (ECMO) system including a blood oxygenator having an outer shell surrounding an oxygenator fiber bundle, a heating element, a closed loop control system, and a temperature sensor. The blood oxygenator defining a blood inlet and a blood outlet. The blood oxygenator has a blood flow path that is bifurcated between a first channel and a second channel. The heating element disposed around or within the outer shell. The closed loop control system configured to regulate the heating element. The temperature sensor is electronically linked to the control system. The heating element extends alongside at least one of the first channel and the second channel.

## Description

### BACKGROUND

The present disclosure relates to extracorporeal membrane oxygenation (ECMO), sometimes also referred to as extracorporeal life support (ECLS), and more particularly to an ECMO system capable of heating blood to a desired temperature.

Patients awaiting or recovering from a heart or lung transplant and patients having conditions putting them at risk for heart or lung failure may be candidates for ECMO therapy. ECMO therapy is designed to address an excess of carbon dioxide and/or lack of oxygen in the blood where the lungs are not healthy and/or the heart cannot pump enough blood around the body. ECMO is thus an extracorporeal technique which replaces these respiratory and cardiac functions in various situations ranging from support needed while separately treating the underlying causes of cardiac arrest to late-stage treatment for heart or lung failure. It may be a therapeutic treatment to provide temporary help when needed.

ECMO therapy operates by drawing deoxygenated blood from the body of a patient in a controlled manner to permit oxygenation and removal of carbon dioxide from the blood. The oxygenated blood is then cycled back into the patient. Generally, ECMO systems include a pump, an oxygenator, an oxygen source, a control center to monitor and control the process, and sometimes a blood heater to bring the blood back up to a desired temperature as it is circulated back into a patient's body.

### BRIEF SUMMARY

In one aspect, the present disclosure relates to blood heating systems that are integrated into extracorporeal membrane oxygenation systems (ECMO systems) that include a blood oxygenator device and a blood pump. These blood heating systems include heating elements that are integrated into or around the blood flow path of an ECMO system to create heated passages made of multiple diameter tubes to divide the blood flow. In particular, these heated passages are disposed around an outer housing of the blood oxygenator device such that the outer housing defines additional tunnels or channels for blood to flow through before it reaches a distribution volute. To improve heating the blood, the heated passages of the outer housing may be split into small passages and split again to create more surface area and thereby increase the heat transfer between heating elements and the blood. In other words, one passage could be split into three or four smaller passages that may be adjacent to or wrapped within heat elements. One heating element or multiple heating elements may be used within or around a passage. In some arrangements, heating elements may be placed in the potting material within the outer housing.

To eliminate size and weight typically associated with fluid-based heating systems, the heating elements consist of a resistive wire embedded in heat conductive resin that is wired to a control system that controls the electrical power applied to the wire. The control system may be a closed loop control system that utilizes temperatures sensors placed along the blood flow path to take temperature measurements used to regulate the power transmitted to the heating elements and thereby control or regulate the heat emitted by the heating elements. For example, a temperature sensor may be placed at an outlet of the outer housing or in a potting material near the outlet of the outer housing. Multiple temperature sensors may be utilized to individually control respective heating elements to maintain even heating throughout the device. Heating elements may be place in multiple locations in and around the oxygenator device, e.g., but not limited to, in or around the blood passageways between the blood oxygenator and the blood pump, embedded in the potting of the fiber bundle of the device, and/or in the inlet of the volute. Alternatively, the heating elements may be a part of a blood heater arranged as a standalone device using either straight, curved, or helical blood channels with heating elements surrounding and/or placed adjacent to the blood channels or passageways, or wrapped concentrically around the blood channels or passageways.

In another aspect, the present disclosure relates to a blood oxygenator that may include an outer shell, a blood flow path, a first heating element, a control system, and a temperature sensor. The outer shell may surround an oxygenator fiber bundle of the blood oxygenator. The blood flow path may be bifurcated between a first channel and a second channel that extend around the outer shell, and the blood flow path may have a blood inlet and a blood outlet. The control system may be configured to regulate heat emitted by the first heating element. The control system may be a closed loop system. The temperature sensor may be electronically linked to the control system. The first heating element may be disposed in or around a portion of the blood flow path. The first heating element may be disposed in an inlet of a volute of the blood oxygenator.

The blood oxygenator may further include a second heating element. The first heating element and the second heating element may be disposed on opposite ends of the outer shell. The first heating element may be embedded in a first potting material, and the second heating element may be embedded in a second potting material. The blood oxygenator may further include a medial channel that extends from an inlet of a volute and disposed between the first and the second channels along the longitudinal axis of the outer shell. The medial blood line may be in fluid communication with both the first and the second channels. The control system may include a software operating system configured to process and/or save blood temperature data to regulate power transmitted to the first heating element. The first heating element may be embedded between the oxygenator fiber bundle and the outer shell. The temperature sensor may be embedded in at least one of the first potting material and the second potting material. The temperature sensor may be disposed at the blood outlet. The first heating element may be a resistive wire encased in a resin. The resin may be a heat conductive resin. The blood inlet may be bifurcated into the first channel and the second channel. The first and the second channels may merge to form the medial channel.

In yet another aspect, the present disclosure relates to a blood oxygenator that may include an outer shell surrounding an oxygenator fiber bundle, and the outer shell may have a blood inlet, a blood outlet, and a volute disposed between the blood inlet and the blood outlet. The blood oxygenator may further include a heating element and a control system. The outer shell may define a blood flow path extending from the blood inlet through the volute. The volute may include a top channel, a bottom channel, and a middle channel disposed between the top and bottom channels along a longitudinal axis of the outer shell. The heating element may be disposed in or around at least a portion of the blood flow path. The top channel, the bottom channel and the middle channel may extend entirely or partially around the circumference of the outer shell. The blood flow path may split at the blood inlet into the top channel and the bottom channel. The top channel and the bottom channel may merge into the middle channel. The middle channel may terminate at an inlet of the volute. The top, the middle and the bottom channels may flow in succession starting with the top channel. The control system may be a closed loop control system that includes at least one temperature sensor. The heating element may be or include a resistive wire embedded in a heat conductive resin. The heating element may include a first heating element and a second heating element disposed on opposite ends of the outer shell. The first heating element may be disposed in the top channel, and the second heating element may be disposed in the bottom channel. The blood oxygenator may further include a third heating element disposed within the middle channel. The blood oxygenator may also include a volute heating element disposed within the volute.

In another aspect, the present disclosure relates to a blood oxygenator that may include an outer shell, a plurality of heating elements, a temperature sensor, a blood flow sensor, and a computer process. The outer shell may be arranged to surround an oxygenator fiber bundle. The outer shell may have a blood inlet, a blood outlet, and a volute disposed between the blood inlet and the blood outlet. The outer shell may define a blood flow path extending from the blood inlet through the volute that includes a top channel, a bottom channel, and a middle channel. The middle channel may be disposed between the top and bottom channels along a longitudinal axis of the outer shell. The computer processor may be connected to the temperature sensor and/or the blood flow sensor and configured to monitor the temperature of blood in the blood oxygenator. The computer processor may be configured to regulate electrical power transmitted to each heating element of the plurality of heating elements. The plurality of heating elements may be disposed at different locations along the blood flow path.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1 is a front perspective view of a blood oxygenator device in accordance with an aspect of the present disclosure;
FIG. 2 is a back perspective view of the blood oxygenator device of FIG. 1;
FIG. 3 is a perspective view of a heated flow path in accordance with an aspect of the present disclosure; and
FIG. 4 illustrates a heating element integrated into a blood oxygenator device in accordance with an aspect of the present disclosure.

### DETAILED DESCRIPTION

The specific examples disclosed herein relate to structural characteristics of various blood oxygenator devices integrated with heating elements designed for the specific goal of improving a patient's ability to ambulate while undergoing ECMO therapy. Examples described herein refer to various types of components and features used in heating systems, power control systems, and blood pumping systems that are designed to integrate with each other. However, the use or non-use of any one of these components or features of these systems is not limited by the use or non-use of the other components or systems disclosed herein.

In one aspect, the present disclosure relates to a blood heater having heating elements that are integrated along a blood flow path, discussed below, of a blood oxygenator device to heat the blood flowing through the device to a desired temperature before it reenters a patient's body.

As described herein, ECMO systems may include a blood heater to bring the patient's blood back up to a desired temperature as it is circulated into the patient's body. Blood heaters are often fluid-based heating systems which require water heater components such as hosing and fittings. These fluid-based heating systems increase the size and weight of ECMO systems and are susceptible to leaking as components wear overtime. Accordingly, to improve patients' ability to ambulate while undergoing ECMO therapy, there remains a need for compact heating mechanisms and methods capable of replacing fluid-based heating systems to reduce the size and weight of ECMO systems and eliminate the risk of fluid leaks. Furthermore, a compact heating mechanism may eliminate the need for additional heat sources when in, and after, surgery on ECMO and/or cardiac bypass.

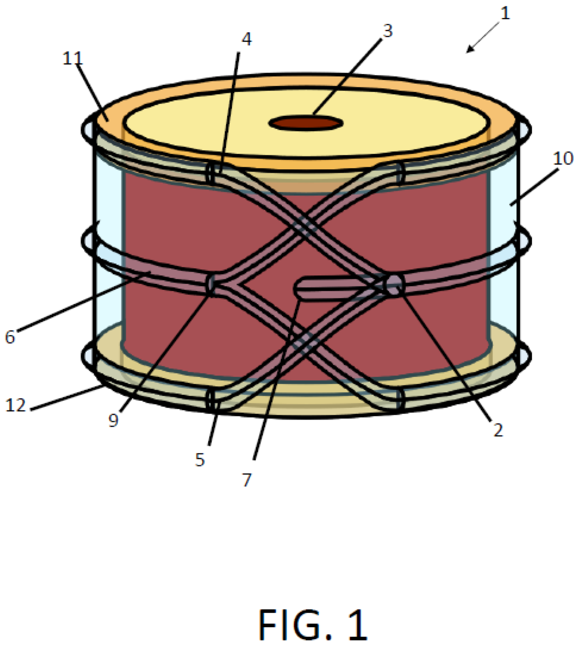

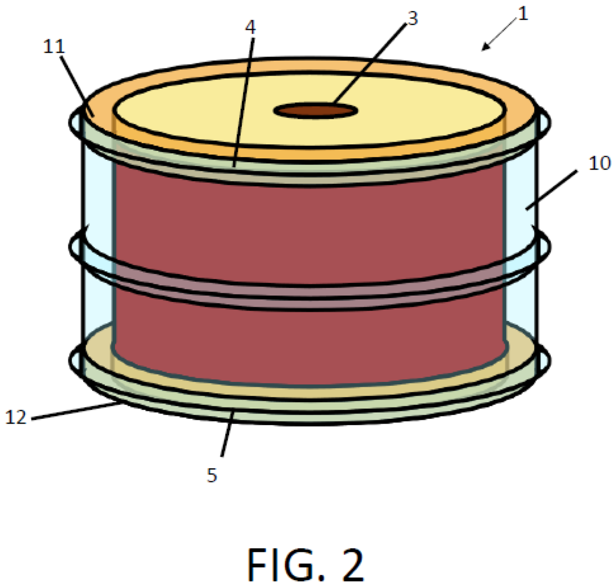

Referring now to the drawings, FIGS. 1-2 show a blood oxygenator device 1 that includes an outer shell 10, a fiber bundle disposed within the outer shell or housing, and a volute (not shown) configured to distribute blood around the fiber bundle similar to the blood oxygenator devices disclosed in U.S. Patent No. 11,065,375, the entire disclosure of which is hereby incorporated herein by reference. Blood oxygenator device 1 defines a blood flow path that flows through an inlet 2 of the device and exits the outer shell at outlet 3. After passing through inlet 2, the blood flow path bifurcates into a top channel 4 and a bottom channel 5 that extend around outer shell 10. It is contemplated that this bifurcation of the blood flow path could take place anywhere between a pump (not shown) and the volute of blood oxygenator device 1. It is further contemplated that top channel 4 and bottom channel 5 may further split into different channels or passageways after the first bifurcation.

Top channel 4 extends around a first end 11 of outer shell 10, and bottom channel 5 extends around a second end 12 of the outer shell, as shown in FIG. 1. Top channel 4 and bottom channel 5 then converge and merge together at wye conjunction 9 to form a middle channel 6 that also extends around outer shell 10 then connects into a distribution inlet 7 where the blood flow path enters the volute (not shown) to be distributed around the fiber bundle. Once the blood flow has been distributed around the fiber bundle, the blood progresses radially from the outer surface of the fiber bundle to a central lumen located at the center of the fiber bundle. From the central lumen, the blood flow path routes up and out through outlet 3 of outer shell 10 where it exits blood oxygenator device 1. Alternatively, instead of the blood flow path bifurcating and running along top channel 4 and bottom channel 5 in parallel (i.e., simultaneously), the blood flow path could first flow through top channel 4 and then through bottom channel 5 before entering the middle channel 6 and volute inlet 7. These channels may be integral to outer shell 10 and run along the outer surface of the outer shell such that the channels are defined by the material of the outer shell.

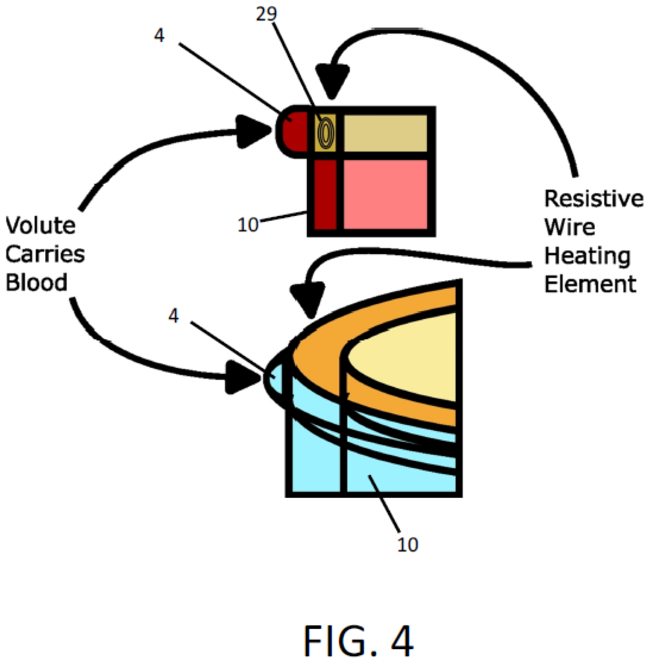

Portions of the blood flow path are heated by a heating element or elements integrated within or around the path. For example, a heating element may be disposed within top channel 4 and/or bottom channel 5 such that blood flows alongside the heating element allowing it to heat up the blood flowing along the path. There may be two separate heating elements such as a top heating element that is disposed in top channel 4 and a bottom heating element that is disposed in bottom channel 5. In some instances, a heating element or elements may be disposed adjacent to the blood flow path of the blood oxygenator device. For example, as shown in FIG. 4, heating element 29 may be disposed next to top channel 4 and/or bottom channel 5 of the blood oxygenator device. In this manner, the heating elements are integrated into the blood oxygenator device and thereby provide a compact and efficient means for heating blood to a desired temperature.

Outer shell 10 may include a first volute and a second volute disposed on opposite ends of the outer shell. The first volute defines a first volute inlet at first end 11, and the second volute defines a second volute inlet at second end 12. The blood flow path may split at inlet 2 into top channel 4 and bottom channel 5 that extend around outer shell 10 and then connect to the first volute inlet and the second volute inlet, respectively. In such instances, heating elements may be disposed within the first volute inlet and the second volute inlet in addition to portions of top channel 4 and bottom channel 5.

In some arrangements, blood oxygenator device 1 may include potting material disposed on opposite ends of the fiber bundle, as disclosed in the '375 Patent. In such instances, the heating element may be embedded in the perimeter of the potting material of blood oxygenator device 1 such that the heating element is adjacent to blood channels within the outer shell of the device. In this manner, the heating element heats up the blood via the potting material as the blood flows therethrough.

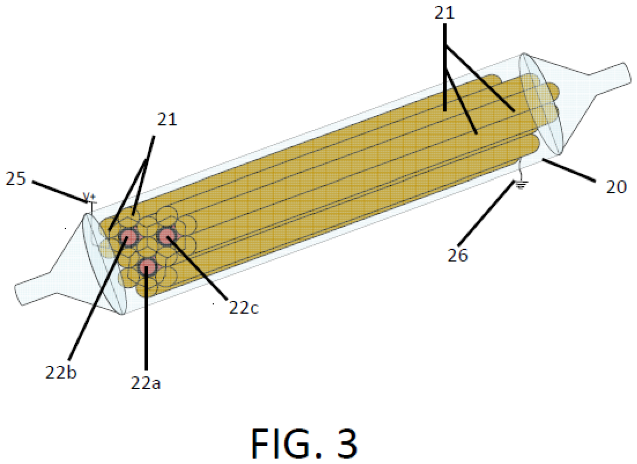

In another arrangement, the blood oxygenator device may include a tubular structure 20 that extends along the blood flow path and has heating elements 21 disposed within the tubular structure. In such instances, the blood flowing into tubular structure 20 is divided up into smaller flow tubes or channels 22a, 22b, 22c which are surrounded by heating elements 21, as shown in FIG. 3. These channels 22a, 22b, 22c may be molded plastic tubes made from the same material as outer shell 10, blood compatible tubing, or simply open space between heating elements 21. Dividing the blood flow with smaller flow tubes or channels 22a, 22b, 22c increases the surface area of the blood flowing through tubular structure 20 to improve heat transfer between the heating elements and the blood. Alternatively, tubular structure 20 may be adjacent to, surrounded by, or wrapped concentrically with heating elements 21. In this manner, blood flowing through the blood oxygenator device 1 can be evenly and efficiently heated back to a desired temperature before it reenters the body.

The heating elements disclosed herein include a resistive wire encased or coated in a heat conductive resin, e.g., but not limited, Vertelis, and connected to an electrical power source and a ground connection (e.g., power source 25 and ground connection 26 shown in FIG. 3). Heating elements may be wired together in series or in parallel. The resistive wire may be made from a variety of metals and/or non-metal materials. For example, the resistive wire may be made from a nickel-chromium alloy and may or may not have a ceramic or fiberglass core.

The heating elements use conductive heating to heat the blood to a User's or Clinician's desired level. This is accomplished by using resistive wire elements to convert electrical power into heat. To prevent developing localized points of high heat, the resistive elements are potted in a heat conductive resin to maintain separation from the blood. The blood flow paths described herein extend through a network of tunnels or passages that are made of the same or similar material as the tubing used to flow blood to the blood oxygenator device and are bonded to the heating elements using the same resin as is used to encapsulate the heating elements. In this manner, the blood oxygenator device can maintain blood compatibility.

The power, e.g., electrical power, applied to the heating element(s) is controlled by a control system that relies on temperature measurements taken from the blood flow path to regulate the applied power to the heating element(s) and thereby regulate the heat emitted from the heating element(s). The control system may be operated with a software system or simply an electronic system that communicate with one or more temperature sensors along the blood flow path. The temperature sensors may be encased in the same or similar heat conductive resin used to coat or encase the heating elements (discussed above). In this manner, the heat applied to the blood may be controlled to provide the desired blood temperature. For example, a desired blood temperature may be entered into the control system, which is then compared to the current temperature of the blood. The current temperature may be taken at a specific location via a sensor or it may be an average of temperature measurements taken at various locations along the blood flow path via a network of temperature sensors. Based on the difference between the desired blood temperature and the current blood temperature, if any, the control system adjusts how much power is transmitted to the heating element(s). The control system may be a closed loop control system that automatically regulates the temperature of the heating element (i.e., regulating the power transmitted to the heating element) by using a feedback controller the relies on temperature measurements obtained from temperatures sensors.

In some arrangements, a temperature sensor may be placed at outlet 3 of the outer shell 10 to collect temperature measurements of the blood as it exits blood oxygenator device 1. These exit temperature measurements are then transmitted to the control system to determine whether the heating element(s) need more or less power to heat the blood to a desired temperature provided by a user or clinician. When the temperature measurements are below the desired temperature, the control system will increase the power applied to the heating element(s). When the temperature measurements are above the desired temperature, the control system may decrease or stop the power applied to the heating element(s). The control system may utilize a software system to regulate how much power is transmitted to the heating element(s). In other instances, the control system may utilize electronic systems to control the power transmitted to the heating element(s). In situations where multiple temperature sensors are use along the blood flow path, these temperature sensors may be positioned near a respective heating element such that the temperature of the blood around that element may be determined.

The control system(s) described herein may include a structure and configuration for executing instructions that implement the operations of a blood oxygenator device (e.g., blood oxygenator device 1) including, but not limited to, regulating power to heat blood flowing through the oxygenator device and monitoring the temperature of the blood therein. The control system may be implemented with an array of multiple logic gates or a general-purpose microprocessor for processing various forms of data including numerical data, textual data, images and/or various other types of data, and may include a single processor or a plurality of processors configured to execute computer applications or programs. For example, the control system may be implemented in the form of at least one of a microprocessor, a central processing unit (CPU), a graphics processing unit (GPU), and/or an accelerated processing unit (APU).

The control system(s) described herein may operate with a memory configured to store various data, instructions, software, mobile applications, computer programs, etc. The memory may be configured separately from or integrally with the control system described herein. The control system may execute instructions stored in the memory to process various calculations and various forms of data including, but not limited to, processing data relating to the temperature and flow of blood passing through a blood oxygenator device. Also, the control system may execute instructions stored in the memory to process various calculations and iterations involving temperature, power supply, flow rate, etc. as discussed herein. For example, the memory may be implemented in the form of a non-volatile memory device such as read only memory (ROM), programmable read only memory (PROM), erasable programmable read only memory (EPROM), electrically erasable programmable read only memory (EEPROM), flash memory, programmable random access memory (PRAM), magnetoresistive random access memory (MRAM), resistive random access memory (RRAM), ferroelectric random access memory (FRAM), etc., or a volatile memory device such as dynamic random access memory (DRAM), static (SRAM), synchronous dynamic (SDRAM), RRAM, hard disk drive (HDD), solid state drive (SSD), flash memory card (e.g., SD, Micro- SD), etc., or may be implemented in the form of a combination thereof.

Additionally, blood heater for the blood oxygenator device may include safety features to help regulate the temperature of a patient's blood. For example, the blood heater may include a control system configured to increase, reduce and/or stop power to the heating elements when a measured blood flow is increased, reduced or stopped to prevent overheating the blood and/or the device. This may be done by integrating blood flow sensors into the blood heater and thereby along the blood flow path to collect and transmit blood flow data used in a control algorithm deployed by the control system for the blood heater. In this manner, blood flowing through the oxygenator device may be maintained at a safe temperature between 37° C and 40° C. In some instances, the blood heater may include a control system having a digital screen configured to display measured blood temperatures for monitoring and logging. In this manner, the medical personnel and patients can readily access and review blood temperature measurements as needed.

It is to be understood that the disclosure set forth herein includes any possible combinations of the particular features set forth above, whether specifically disclosed herein or not. For example, where a particular feature is disclosed in the context of a particular aspect, embodiment, arrangement, or configuration, that feature can also be used to the extent possible, in combination with and/or in the context of other particular aspects, embodiments, arrangements, and configurations of the technology, and in the technology in general.

Furthermore, although the technology here has been described with reference to particular features and figures, it is to be understood that these features are merely illustrative of the principles and applications of the present technology. It is therefore to be understood that numerous modifications, including changes in the sizes of the various features described herein, may be made to the illustrative arrangement and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. A blood oxygenator, comprising:
an outer shell surrounding an oxygenator fiber bundle;
a blood flow path that is bifurcated between a first channel and a second channel that extend around the outer shell, the blood flow path having a blood inlet and a blood outlet;
a first heating element;
a control system configured to regulate heat emitted by the first heating element; and
a temperature sensor electronically linked to the control system,
wherein the first heating element is disposed in or around a portion of the blood flow path.

2. The blood oxygenator of claim 1, wherein the first heating element is disposed in an inlet of a volute of the blood oxygenator.

3. The blood oxygenator of any one of claims 1 or 2, further comprising a second heating element, wherein the first heating element and the second heating element disposed on opposite ends of the outer shell such that the first heating element is embedded in a first potting material, and the second heating element is embedded in a second potting material.

4. The blood oxygenator of any one of claims 1-3, further comprising a medial channel that extends from an inlet of a volute and is disposed between the first and the second channels along the longitudinal axis of the outer shell, wherein the medial blood line is in fluid communication with both the first and the second channels.

5. The blood oxygenator of any one of claims 1-4, wherein the control system includes a software operating system configured to process blood temperature data to regulate power transmitted to the first heating element.

6. The blood oxygenator of any one of claims 1-5, wherein the first heating element is embedded between the oxygenator fiber bundle and the outer shell.

7. The blood oxygenator of any one of claims 1-6, wherein the temperature sensor is embedded in at least one of the first potting material and the second potting material.

8. The blood oxygenator of any one of claims 1-7, wherein the temperature sensor is disposed at the blood outlet.

9. The blood oxygenator of any one of claims 1-8, wherein the first heating element is a resistive wire encased in a resin.

10. The blood oxygenator of claim 9, wherein the resin is a heat conductive resin.

11. The blood oxygenator of claim 4, wherein the blood inlet is bifurcated into the first channel and the second channel.

12. The blood oxygenator of claim 11, wherein the first and the second channels merge to form the medial channel.

13. The blood oxygenator of any one of claims 1-12, further comprising a third heating element disposed within the middle channel.

14. The blood oxygenator of any one of claims 2 and 3-13 depending from claim 2, further comprising a volute heating element disposed within the volute.

15. The blood oxygenator of any one of claims 1-14, wherein the control system is a closed loop control system that includes at least one blood flow sensor.
